# EUROPEAN PATENT SPECIFICATION

(11) **EP 3 729 092 B1**
(45) Date of publication and mention of the grant of the patent: **25.11.2026**
(21) Application number: 18833201.9
(22) Date of filing: 19.12.2018
(51) Int. Cl.: G01N 33/564, C07K 16/00, G01N 33/5752, C07K 16/18

(54) **TUMSTATIN ASSAY**
TUMSTATIN-TEST
DOSAGE DE TUMSTATINE

(30) Priority: 20.12.2017 GB 201721387
(43) Date of publication of application: 28.10.2020
(73) Proprietor: Nordic Bioscience A/S, 2730 Herlev (DK)
(72) Inventor: NIELSEN, Signe, Holm, 2400 København NV (DK); KARSDAL, Morten, 2100 København Ø (DK); GENOVESE, Federica, 2000 Frederiksberg (DK)
(74) Representative: Beck Greener LLP
(86) International application number: PCT/EP2018/085855
(87) International publication number: WO 2019/121925

(56) References cited:
- C. M. BORZA ET AL: "Human Podocytes Adhere to the KRGDS Motif of the 3 4 5 Collagen IV Network", JOURNAL OF THE AMERICAN SOCIETY OF NEPHROLOGY., vol. 19, no. 4, 18 March 2008 (2008-03-18), US, pages 677 - 684, XP055567230, ISSN: 1046-6673, DOI: 10.1681/ASN.2007070793
- PENADES J R ET AL: "CHARACTERIZATION AND EXPRESSION OF MULTIPLE ALTERNATIVELY SPLICED TRANSCRIPTS OF THE GOODPASTURE ANTIGEN GENE REGION. GOODPASTURE ANTIBODIES RECOGNIZE RECOMBINANT PROTEINS REPRESENTING THE AUTOANTIGEN AND ONE OF ITS ALTERNATIVE FORMS", EUROPEAN JOURNAL OF BIOCHEMISTRY, WILEY-BLACKWELL PUBLISHING LTD, GB, vol. 229, no. 3, 1 May 1995 (1995-05-01), pages 754 - 760, XP000938485, ISSN: 0014-2956, DOI: 10.1111/J.1432-1033.1995.TB20524.X
- F REVERT ET AL: "Phosphorylation of the Goodpasture Antigen by Type A Protein Kinases", THE JOURNAL OF BIOLOGICAL CHEMISTRY, vol. 270, no. 2, 1 January 1995 (1995-01-01), pages 13254 - 13261, XP055566955
- SIGNE HOLM NIELSEN ET AL: "Tumstatin, a Matrikine Derived from Collagen Type IV[alpha]3, is Elevated in Serum from Patients with Non-Small Cell Lung Cancer", TRANSLATIONAL ONCOLOGY, vol. 11, no. 2, 1 April 2018 (2018-04-01), United States, pages 528 - 534, XP055566836, ISSN: 1936-5233, DOI: 10.1016/j.tranon.2018.02.005

## Description

### Field of the Invention

The present invention relates to an assay for detecting Tumstatin, and its use in evaluating lung cancers, such as non-small cell lung cancer (NSCLC), chronic kidney disease (CKD), such as CKD resulting from diabetes, lupus nephritis (LN) and systemic lupus erythematosus (SLE).

### Background Art

The basement membrane (BM) is a specialized extracellular matrix (ECM), which functions as a scaffold for epithelial and endothelial cells, and acts as a barrier between tissues (1,2). Two of the main BM proteins are collagen type IV and laminin, which together form a distinct network linked together by nidogen and heparin sulphate proteoglycans (2-5). Collagen type IV has six different α-chains, α1-6, which form the heterotrimers expressed in the mammalian BMs(6). The α3 chain of collagen type IV (COL4α3), has been described to have restricted distribution across BMs and is generally found in the lungs and kidneys (7). The structural role of COL4α3 is illustrated by clinical manifestations of Alport's syndrome, Goodpasture's syndrome, and several autoimmune disease targeting the lungs and kidneys' BM. These diseases are characterized by damage to COL4α3 by either mutations or immune attacks which cause leakage of the BM (8-11). The BM serves as a barrier for cell invasion. Breaching of the BM and loss of BM integrity are associated with an invasive cancer phenotype (12). Cancer biomarkers associated with breach and disorganization of the BM are therefore needed.

Tumstatin (TUM) is a 28-kDa fragment of COL4α3 that binds to endothelial cells via the αvβ3 integrin (13). It is a matrikine generated by matrix metalloproteinase-9 (MMP-9), and it is known to keep pathological angiogenesis and tumour growth in check (13-15). MMP-9 is needed to cleave tumstatin from COL4α3 so that tumstatin can function as a protective matrikine. Lack of MMP-9 accelerates tumour growth in MMP-9 knockout mice. High levels of COL4α3 mRNA were associated with a poor prognosis in patients with lung cancer (15,16). Several studies have speculated that matrikines may be potential biomarkers with therapeutical potential (10,17-19).

Revert et al (21) developed an antibody against the GPpep1 peptide consisting of residue 3-23 of the Goodpastures antigen. This antibody was used by Borza et al (22) to investigate the role of the KRGDS motif within the protein. Penades et al (23) also developed antibodies against the GPpep1 peptide.

Luo et al (20) developed a sandwich ELISA for the quantification of COL4α3/tumstatin in human serum and tissue extracts. However, no significant difference was found in patients with lung carcinoma without metastatic disease compared to healthy controls, with the only relevant finding being a decreased level of COL4α3/tumstatin in patients with metastatic lung carcinoma compared to patients without metastatic lung carcinoma. Thus, whilst the ELISA of Luo may be able to quantify COL4α3/tumstatin in human serum and tissue extracts, the diagnostic utility of that assay is shown to be somewhat limited.

### Summary of the Invention

The present inventors have now developed a tumstatin assay that demonstrates excellent diagnostic utility.

The invention is set out in the appended claims.

Thus, in a first aspect the present invention relates to an immunoassay method for quantifying peptides having an N-terminus amino acid sequence PGLKGKRGDS (SEQ ID NO:1) in a patient biofluid sample, said method comprising contacting said patient biofluid sample with a monoclonal antibody specifically reactive with said N-terminus amino acid sequence PGLKGKRGDS (SEQ ID NO:1), and determining the amount of binding between said monoclonal antibody and said N-terminus amino acid sequence.

The monoclonal antibody does not specifically recognise or bind an N-extended elongated version of said N-terminus amino acid sequence or an N-truncated shortened version of said N-terminus amino acid sequence. In this regard "N-extended elongated version of said N-terminus amino acid sequence" means one or more amino acids extending beyond the N-terminus of the sequence H₂N-PGLKGKRGDS (SEQ ID NO:1). For example, if the N-terminal amino acid sequence H2N-PGLKGKRGDS (SEQ ID NO:1) was elongated by a leucine residue then the corresponding "N-extended elongated version" would be H₂N-LPGLKGKRGDS(SEQ ID NO:2). Similarly, "N-truncated shortened version of said N-terminus amino acid sequence" means one or more amino acids removed from the N-terminus of the sequence H₂N-PGLKGKRGDS(SEQ ID NO:1). For example, if the N-terminal amino acid sequence H₂N- PGLKGKRGDS (SEQ ID NO:1) was shortened by one amino acid residue then the corresponding "N-truncated shortened version" would be H₂N- GLKGKRGDS (SEQ ID NO:3).

In a second aspect, the present invention relates to a method of immunoassay for detecting lung cancer in a patient, the method comprising contacting a patient biofluid sample with a monoclonal antibody specifically reactive with an N-terminus amino acid sequence PGLKGKRGDS (SEQ ID NO:1), determining the amount of binding between said monoclonal antibody and peptides comprising said N-terminus amino acid sequence, and correlating said amount of binding with i) values associated with normal healthy subjects and/or ii) values associated with known lung cancer severity and/or iii) values obtained from said patient at a previous time point and/or iv) a predetermined cut-off value.

The lung cancer may be, but is not limited to, non-small cell lung cancer (NSCLC).

The predetermined cut-off value may be at least 2.00 ng/mL, preferably at least 2.30 ng/mL, more preferably at least 2.60 ng/mL, and most preferably at least 3.00 ng/mL. In this regard, through the combined use of various statistical analyses it has been found that a measured amount of binding between the monoclonal antibody (described above) and the N-terminus biomarker of at least 2.00 ng/mL or greater may be determinative of the presence of lung cancer, such as NSCLC. By having a statistical cutoff value of at least 2.00 ng/mL, preferably at least 2.30 ng/mL, more preferably at least 2.60 ng/mL, and most preferably at least 3.00 ng/mL, it is possible to utilise the method of the invention to diagnose lung cancer with a high level of confidence. Or, in other words, applying the statistical cutoff value to the method of the invention is particularly advantageous as it results in a standalone diagnostic assay; i.e. it removes the need for any direct comparisons with healthy individuals and/or patients with known disease severity in order to arrive at a diagnostic conclusion. This may also be particularly advantageous when utilising the assay to evaluate patients that already have medical signs or symptoms that are generally indicative of lung cancer (e.g. as determined by a physical examination and/or consultation with a medical professional) as it may act as a quick and definitive tool for corroborating the initial prognosis and thus potentially remove the need for more invasive procedures, such as endoscopy and/or biopsy, and expedite the commencement of a suitable treatment regimen. In the particular case of lung cancer, an expedited conclusive diagnosis may result in the disease being detected at an earlier stage, which may in turn improve overall chances of survival.

The monoclonal antibody used in the above method does not specifically recognise or bind an N-extended elongated version of said N-terminus amino acid sequence or an N-truncated shortened version of said N-terminus amino acid sequence.

In a third aspect, the present invention relates to a method of immunoassay for detecting chronic kidney disease (CKD) in a patient, the method comprising contacting a patient biofluid sample with a monoclonal antibody specifically reactive with an N-terminus amino acid sequence PGLKGKRGDS (SEQ ID NO:1), determining the amount of binding between said monoclonal antibody and peptides comprising said N-terminus amino acid sequence, and correlating said amount of binding with i) values associated with normal healthy subjects and/or ii) values associated with known CKD severity and/or iii) values obtained from said patient at a previous time point and/or iv) a predetermined cut-off value.

The CKD may be, but is not limited to, CKD resulting from systemic lupus erythematosus (SLE), lupus nephritis (LN) or diabetes.

The predetermined cut-off value may be at least 2.00 ng/mL, preferably at least 2.30 ng/mL, more preferably at least 2.60 ng/mL, and most preferably at least 3.00 ng/mL.

The monoclonal antibody does not specifically recognise or bind an N-extended elongated version of said N-terminus amino acid sequence or an N-truncated shortened version of said N-terminus amino acid sequence.

In a fourth aspect, the present invention relates to a method of immunoassay for detecting systemic lupus erythematosus (SLE) or lupus nephritis (LN) in a patient, the method comprising contacting a patient biofluid sample with a monoclonal antibody specifically reactive with an N-terminus amino acid sequence PGLKGKRGDS (SEQ ID NO:1), determining the amount of binding between said monoclonal antibody and peptides comprising said N-terminus amino acid sequence, and correlating said amount of binding with i) values associated with normal healthy subjects and/or ii) values associated with known SLE or LN severity and/or iii) values obtained from said patient at a previous time point and/or iv) a predetermined cut-off value.

The predetermined cut-off value may be at least 2.00 ng/mL, preferably at least 2.30 ng/mL, more preferably at least 2.60 ng/mL, and most preferably at least 3.00 ng/mL.

The monoclonal antibody does not specifically recognise or bind an N-extended elongated version of said N-terminus amino acid sequence or an N-truncated shortened version of said N-terminus amino acid sequence.

In all of the above described methods according to any of the first to fourth aspects of the invention, the patient biofluid sample may be, but is not limited to, blood, urine, synovial fluid, serum or plasma. In certain preferred embodiments the biofluid sample may be urine or serum. In methods of immunoassay for detecting chronic kidney disease (CKD), systemic lupus erythematosus (SLE) or lupus nephritis (LN), it may in particular be preferred that the biofluid sample is urine.

In a fifth aspect, the present invention relates to a monoclonal antibody specifically reactive with an N-terminus amino acid sequence PGLKGKRGDS (SEQ ID NO:1).

The monoclonal antibody does not specifically recognise or bind an N-extended elongated version of said N-terminus amino acid sequence or an N-truncated shortened version of said N-terminus amino acid sequence.

In a sixth aspect, the present invention relates to an assay kit comprising a monoclonal antibody specifically reactive with an N-terminus amino acid sequence PGLKGKRGDS (SEQ ID NO:1), and at least one of:
- a streptavidin coated well plate
- a biotinylated peptide PGLKGKRGDS-L-Biotin (SEQ ID NO:6), wherein L is an optional linker
- a secondary antibody for use in a sandwich immunoassay
- a calibrator peptide comprising the sequence PGLKGKRGDS (SEQ ID NO:1)
- an antibody biotinylation kit
- an antibody HRP labeling kit
- an antibody radiolabeling kit
- an assay visualization kit

The monoclonal antibody does not specifically recognise or bind an N-extended elongated version of said N-terminus amino acid sequence or an N-truncated shortened version of said N-terminus amino acid sequence.

The monoclonal antibody described supra and/or included in the assay kit may be raised against a synthetic peptide having the amino acid sequence PGLKGKRGDS (SEQ ID NO:1).

### Definitions

As used herein the term "N-terminus" refers to the extremity of a polypeptide, i.e. at the N-terminal end of the polypeptide, and is not to be construed as meaning in the general direction thereof.

As used herein the term, the term "competitive ELISA" refers to a competitive enzyme-linked immunosorbent assay and is a technique known to the person skilled in the art.

As used herein the term "sandwich immunoassay" refers to the use of at least two antibodies for the detection of an antigen in a sample, and is a technique known to the person skilled in the art.

As used herein the term "amount of binding" refers to the quantification of binding between antibody and biomarker, which said quantification is determined by comparing the measured values of biomarker in the biofluid samples against a calibration curve, wherein the calibration curve is produced using standard samples of known concentration of the biomarker. In the specific assay disclosed herein which measures in biofluids the N-terminus biomarker having the N-terminus amino acid sequence PGLKGKRGDS (SEQ ID NO:1), the calibration curve is produced using standard samples of known concentration of the calibration peptide PGLKGKRGDS (SEQ ID NO:1). The values measured in the biofluid samples are compared to the calibration curve to determine the actual quantity of biomarker in the sample. The present invention utilises spectrophotometric analysis to both produce the standard curve and measure the amount of binding in the biofluid samples; in the Examples set out below the method utilises HRP and TMB to produce a measurable colour intensity which is proportional to the amount of binding and which can be read by the spectrophotometer. Of course, any suitable analytical method could also be used.

As used herein the "cut-off value" means an amount of binding that is determined statistically to be indicative of a high likelihood of a disease (e.g. lung cancer, such as NSCLC, or chronic kidney disease, systemic lupus erythematosus, or lupus nephritis), in a patient, in that a measured value of biomarker in a patient sample that is at or above the statistical cutoff value corresponds to at least a 70% probability, preferably at least an 80% probability, preferably at least an 85% probability, more preferably at least a 90% probability, and most preferably at least a 95% probability of the presence or likelihood of a disease (e.g. lung cancer, such as NSCLC, or chronic kidney disease, systemic lupus erythematosus, or lupus nephritis).

As used herein the term "values associated with normal healthy subjects and/or values associated with known disease severity" means standardised quantities of Tumstatin determined by the method described supra for subjects considered to be healthy, i.e. without a disease (e.g. without lung cancer, such as NSCLC, or chronic kidney disease, systemic lupus erythematosus, or lupus nephritis), and/or standardised quantities of Tumstatin determined by the method described supra for subjects known to have a disease (e.g. lung cancer, such as NSCLC, or chronic kidney disease, systemic lupus erythematosus, or lupus nephritis), of a known severity.

As used herein, "TUM ELISA" refers to the specific competitive ELISA disclosed herein which quantifies in a sample the amount peptides having the N-terminus amino acid sequence PGLKGKRGDS (SEQ ID NO:1).

### Figures

**Figure 1****.** TUM ELISA showing a typical standard curve and native reactivity against human serum and human urine. The standard peptide was 2-fold diluted starting from 20 ng/mL. The samples were run from undiluted and up to 8-fold dilution as indicated.
**Figure 2****.** Assay specificity. Reactivity to the standard peptide (PGLKGKRGDS; SEQ ID NO:1), the elongated peptide (LPGLKGKRGDS; SEQ ID NO:2), the truncated peptide (GLKGKRGDS; SEQ ID NO:3) and a non-sense peptide (LRSKSKKFRR; SEQ ID NO:4) was tested in the TUM assay.
**Figure 3****.** Results from Cohort 1. Serum TUM levels were assessed in healthy controls (n=8), patients with IPF (n=7), COPD (n=8) and NSCLC (n=8). Data was analyzed using a Kruskal-Wallis test adjusted for Dunn's multiple comparisons test. Data are presented as Tukey box plots. Significance levels: *: p<0.05 and **: p<0.001.
**Figure 4****.** Results from cohort 2. Serum TUM levels were assessed in healthy controls (n=20) and NSCLC (n=40). Data was analyzed using a Mann Whitney t-test. Data are presented as Tukey box plots. Significance levels: *: p<0.05.
**Figure 5****.** Levels of TUM in urine (ng/mg Creatinine) and serum (ng/ml) samples from healthy individuals and from patients with lupus nephritis (LN). **p<0.01.
**Figure 6****.** Levels of TUM in serum samples (ng/ml) from healthy individuals and patients with systemic lupus erythematosus (SLE) .
**Figure 7****.** Levels of TUM in urine samples in a rat model of diabetic kidney disease.

### Examples

The presently disclosed embodiments are described in the following Examples, which are set forth to aid in the understanding of the disclosure, and should not be construed to limit in any way the scope of the disclosure as defined in the claims which follow thereafter. The following examples are put forth so as to provide those of ordinary skill in the art with a complete disclosure and description of how to make and use the described embodiments, and are not intended to limit the scope of the present disclosure nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

In the following examples, the following materials and methods were employed.

### Materials and methods

All reagents used for the experiments were high quality standards from companies such as Sigma Aldrich (St. Louis, MO, USA) and Merck (Whitehouse Station, NJ, USA). The synthetic peptides used for immunization and assay development were purchased from the Genscript (New Jersey, USA).

### Generation of monoclonal antibodies

The amino acid sequence 1426'PGLKGKRGDS'1436 (SEQ ID NO:1) is located in the α3 chain of type IV collagen. This sequence is generated towards human tumstatin, and has a mismatch in amino acid (AA) position 6 in rats and a mismatch in AA position 5 in mice. Immunization was initiated by subcutaneous injection of 200 uL emulsified antigen and 50 ug immunogenic peptide (PGLKGKRGDS-GGC-KLH; SEQ ID NO:5) in 4-6 weeks old Balb/C mice using Freund's incomplete adjuvant. The immunizations were repeated every 2^{nd} week until stable serum antibody titer levels were reached. The mouse with the highest serum titer was selected for fusion and rested for a month. Subsequently, the mouse was boosted intravenously with 50 ug immunogenic peptide in 100 uL 0.9% NaCl solution three days before isolation of the spleen for cell fusion. To produce hybridoma cells, the mouse spleen cells were fused with SP2/0 myeloma cells as described by Gefter et al. The hybridoma cells were cloned in culture dishes using the semisolid medium method. The clones were then plated into 96-well microtiter plates for further growth, and the limiting dilution method was applied to promote monoclonal growth. Indirect ELISA performed on streptavidin-coated plates was used for the screening of supernatant reactivity. PGLKGKRGDS-K-Biotin (SEQ ID NO:6) was used as the screening peptide, while the standard peptide PGLKGKRGDS (SEQ ID NO:1) was used for further test of specificity of clones. Supernatant was collected from the hybridoma cells, and purified using HiTrap affinity columns GE Healthcare Life Science, Little Chalfront, Buckinghamshire, UK) according to manufacturer's instructions. The production of monoclonal antibodies performed in mice was approved by the National Authority (The Animal Experiments Inspectorate) under approval number 2013-15-2934-00956. All animals were treated according to the guidelines for animal welfare.

### Clone characterization

Native reactivity and peptide affinity for the standard peptide were assessed using human serum and human urine purchased from a commercial supplier (Valley Biomedical, VA 22602, USA). Antibody specificity was tested in a preliminary assay using truncated (GLKGKRGDS; SEQ ID NO:3) and elongated peptides (LPGLKGKRGDS; SEQ ID NO:2). The isotype of the monoclonal antibody was determined using the Clonotyping System-HRP kit, cat. 5300-05 (Southern Biotech, Birmingham, AL, USA).

### TUM ELISA

The TUM competitive ELISA procedure was as follows: 96-well streptavidin-coated ELISA plates (Roche, cat. 11940279) were coated with 10 ng/mL biotinylated peptide PGLKGKRGDS-K-Biotin (SEQ ID NO:6) dissolved in assay buffer (25 mM Tris-BTB 2g. NaCl/L, pH 8.0, 100 µL/well) and incubated for 30 min at 20° C in the dark with 300 rpm shaking. Plates were washed five times in washing buffer (20 mM TRIS, 50 mM NaCl, pH 7.2). Subsequently, 20 µL of standard peptide or sample were added to appropriate wells, followed by 100 µL of 7 ng/mL horseradish peroxidase (HRP) labeled monoclonal antibody solution. The plates were incubated for 1 hour at 20 °C with shaking, and subsequently washed in washing buffer. Finally, 100 µL 3,3',5,5-tetramethylbenzinidine (TMB) (Kem-En-Tec cat. 438OH) was added, and incubated for 15 min at 20 °C. To stop the enzyme reaction of TMB, 100 µL of stopping solution (1% H₂SO₄) was added. The plate was analyzed by an ELISA reader at 450 nm with 650 nm as reference (Molecular Devices, VersaMax, CA, USA). A standard curve was performed by serial dilution of the standard peptide and plotted using a 4-parametric mathematical fit model. Standard concentrations were 0, 0.3125, 0.625, 1.25, 2.5, 5, 10, and 20 ng/mL. Each plate included five kit controls to monitor inter-assay variation. All samples were measured within the range of the assay, and all samples below lower limit of measurement range (LLMR) were reported as the value of LLMR.

### Technical evaluation

A twofold dilution of four human serum and human urine samples was used to assess the linearity. The linearity was calculated as a percentage of recovery of the undiluted sample.

Antibody specificity was calculated as percentage of signal inhibition by 2-fold diluted standard peptide (PGLKGKRGDS; SEQ ID NO:1), elongated peptide (LPGLKGKRGDS; SEQ ID NO:2), truncated peptide (GLKGKRGDS; SEQ ID NO:3) and non-sense peptide (LRSKSKKFRR; SEQ ID NO:4). The lower limit of detection (LLOD) was estimated from 21 determinations of the lowest standard (buffer). LLOD was calculated as mean - 3* standard deviation (SD). Upper limit of detection (ULOD) was determined as the mean ± 3*SD of 10 measurements of Standard A. The intra- and inter-assay variation was determined by 10 independent runs of five quality control (QC) and two kit controls run in double determinations. Accuracy of the assay was measured in healthy human serum/urine samples spiked with standard peptide and a serum/urine sample with a known high Tumstatin concentration, and calculated as the percentage recovery of serum/urine in buffer. Following, spiking recovery was determined by calculating the percentage recovery of spiked serum in buffer. Interference was measured in healthy human serum spiked with either biotin (low = 30 ng/ml, high = 90 ng/ml), hemoglobin (low = 0.155 mM, high = 0.310 mM), or lipids (low = 4.83 mM, high = 10.98 mM). The interference was calculated as the percentage recovery of the analyte in non-spiked serum. Furthermore, a human anti-mouse antibody (HAMA) panel was used to study the interference. Five healthy human serum samples were added to the HAMA panel. These were analyzed with and without 5% Liq II in the dilution buffer. Salt interference was tested by measuring salt samples with a concentration of 8.14 g/L NaCl at pH 7.0 and 8.0. To define the standard concentration of Tumstatin, the normal range was determined by analyzing 32 healthy human serum samples in relation to age and gender of the sample donors. Lower limit of measurement range (LLMR) and upper limit of measurement range (ULMR) was calculated based on the 10 individual standard curves from the intra- and inter-assay variation. The analyte stability was determined for three healthy human serum samples which were incubated at either 4 or 20 °C for 2, 4 and 24 hours respectively. The stability of the samples was evaluated by calculating the percentage variation in proportion to the sample kept at -20 °C (0 hour sample). Furthermore, the analyte stability was determined for three healthy human serum samples, exposed to four freeze and thaw cycles. To assess the stability of the analyte, the percentage of recovery was calculated of a sample undergone only one freeze/thaw cycle.

### Biological validation of TUM as a biomarker for lung cancer

TUM was measured in serum samples from two different cohorts. Both cohorts were obtained from the commercial vendor Proteogenex (Culver City, CA, USA). Cohort 1 included patients diagnosed with IPF, COPD, non-small cell lung cancer (NSCLC) and colonoscopy-negative controls with no symptomatic or chronic disease. Patient demographics are shown in Table 1. Cohort 2 included patients diagnosed with NSCLC in cancer stage I, II, III and IV together with colonscopy-negative controls with no symptomatric or chronic disease. Patient demographics of this cohort can be found in Table 2.

**Table 1. Patient demographics of cohort 1. Data is presented as mean (SD) unless otherwise stated. Comparison of age, gender and BMI was performed using Kruskal-Wallis adjusted for Dunn's multiple comparisons test, while comparison of FEV₁% of predicted value and FEV₁/FVC ratio % were calculated using the Mann-Whitney unpaired t-test. P-values below 0.05 were considered significant. Abbreviations: BMI; body mass index; IPF; idiopathic pulmonary fibrosis, COPD; chronic obstructive pulmonary disease, FEV1, forced expiratory volume in one second, FVC, forced vital capacity.**

| | **Healthy controls** (n=8) | **IPF** (n=7) | **COPD** (n=8) | **NSCLC** (n=8) | **p-value** |
|---|---|---|---|---|---|
| Age | 54.88 (7.85) | 74.13 (8.36) | 75.38 (1.69) | 60.50 (9.32) | <0.001 |
| Male, n (%) | 6 (75%) | 4 (57%) | 4 (50%) | 7 (87.5%) | 0.102 |
| BMI | 26.25 (1.27) | 25.79 (1.58) | 27.24 (1.84) | N/A | 0.170 |
| FEV₁% of predicted value | - | 64.38 (3.42) | 61.5 (7.19) | - | 0.634 |
| FEV₁/FVC ratio % | - | 76.00 (1.51) | 58.38 (15.20) | - | 0.016 |

**Table 2. Patient demographics of cohort 2. Data is presented as mean (SD) unless otherwise stated. Comparison of age, gender and BMI was performed using a Mann-Whitney t-test. P-values below 0.05 were considered significant. Abbreviations: BMI; body mass index.**

| | **Healthy controls** (n=20) | **NSCLC** (n=40) | **p-value** |
|---|---|---|---|
| Age | 61.85 (1.95) | 61.93 (2.14) | 0.593 |
| Male, n (%) | 10 (50%) | 20 (50%) | 1.000 |
| BMI | 26.14 (2.67) | 25.55 (4.23) | 0.533 |

### Statistical analysis

Levels of TUM in serum samples was compared using Kruskal-Wallis adjusted for Dunn's multiple comparisons test (non-paramteric data). Results are presented as Mean±Standard Error of Mean (SEM).

The diagnostic power of TUM was investigated by an area under the receiver operating characteristics (AUROC) curve. Statistical analysis and graphs were performed using GraphPad Prism version 7 (GraphPad Software, Inc., CA, USA).

### Biological validation-TUM as a biomarker for CKD, SLE and LN

TUM was measured in two different patient cohorts. Cohort 1 (18 patients) included individuals with lupus nephritis (LN) and healthy controls, with TUM levels being measured in both serum and urine samples. Cohort 2 (126 patients) included individuals with systemic lupus erythematosus (SLE) and healthy controls, with TUM levels being measured in serum samples only. The patient demographics for Cohort 1 are shown below in Table 3.

**Table 3. Cohort no.1: 18 patients with lupus nephritis (serum and urine)**

| | N or mean | Min-Max |
|---|---|---|
| Gender (male) | 4 | |
| Age | 40 | 17-62 |
| eGFR | 87 | 17.4-165.0 |
| Proteinuria (g/day) | 2.8 | 0-13.9 |

Additionally, TUM was measured in a rat model of diabetic kidney disease. Sprague-Dawley rats (n=8) were injected with streptozocin (STZ) in the tail vein to induce diabetes, and the rats were considered diabetic if their blood glucose was stable above 15 mmol L-1 after 48 hours. After 2 weeks, STZ-treated rats underwent ischemic reperfusion injury (IRI). Control rats (n=7) received a sham operation. Urine samples were taken from the rats at days 0, 1, 5 and 8 after the operation (IRI or sham), and the levels of TUM in the urine samples were measured.

### Results

### Clone characterization

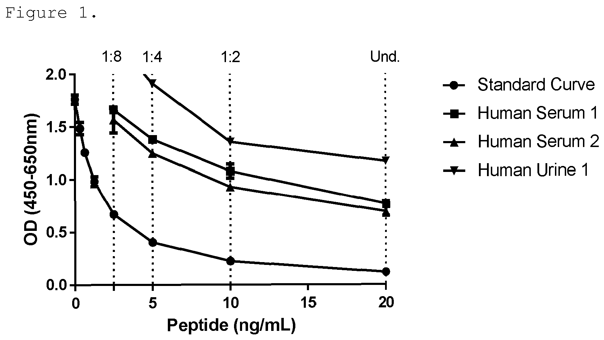

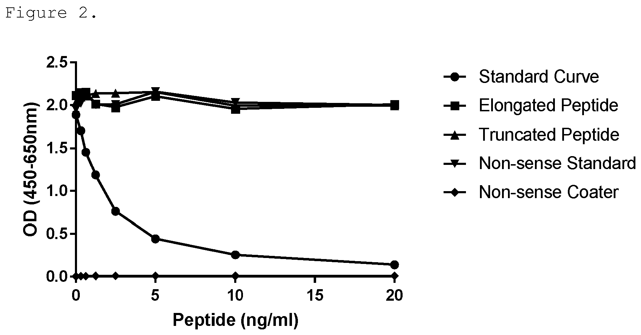

The best antibody producing hybridomas were screened for reactivity towards the standard peptide and native material in the competitive ELISA. Based on the reactivity, the clone NBH134#102-3GF was chosen for assay developed and determined to be the IgG1 subtype. Native reactivity was observed in human serum and urine (Figure 1), while no reactivity was found towards the elongated peptide, truncated peptide, non-sense standard peptide and non-sense coater (Figure 2).

### Technical evaluation of the TUM ELISA assay

A series of technical validations were performed to evaluate the TUM ELISA assay. A summary of the validation data can be found in Table 4. The measurement range (LLMR-ULMR) of the assay was determined to 0.26-9.92 ng/mL. The inter- and intra-variation was 8.04% and 10.96 % respectively. Linearity of the human samples was observed from undiluted to 1:4 for human serum, and undiluted to 1:2 for human urine. Spiking recover tog standard peptide in human serum, and human serum in human serum resulted in a mean recovery of 90 % and 99%, respectively. Neither hemoglobin, lipids nor biotin interfered with measurements of the TUM analyte in human serum. The stability of the analyte was acceptable during both prolonged storage of human serum samples at 4°C and 20°C (102.4% and 80.1%) and during freeze/thaw cycles (80.8%).

**Table 4. Technical validation data of the TUM ELISA assay**

| **Technical validation test** | **TUM** |
|---|---|
| IC50 | 1.6 ng/mL |
| Detection range | 0.26-9.92 ng/mL |
| Intra-assay variation¹ | 8.04 % |
| Inter-assay variation¹ | 10.96 % |
| Dilution recovery in human serum¹ | 89% |
| Dilution recovery in human urine¹ | 98% |
| Analyte recovery 24h, 4°C/20°C¹ | 102.4%/80.1% |
| Hemoglobin recovery, low/high¹ | 100% /100% |
| Lipemia recovery, low/high¹ | 100% /100 % |
| Biotin recovery, low/high¹ | 120% /106% |
| Salt recovery, pH 6.0/pH 7.0/pH 8.0² | 97 % |
| Spiking recovery (peptide in serum)¹ | 90% |
| Spiking recovery (serum in serum)¹ | 99% |
| Analyte recovery, 3 freeze/thaw cycles¹ | 80.8% |

| | |
|---|---|
| ¹Percentages are reported as mean, ²Average recovery after salt interference. | |

### Biological evaluation - TUM as a biomarker for lung cancer

TUM was measured in two different cohorts; cohort 1 and cohort 2.

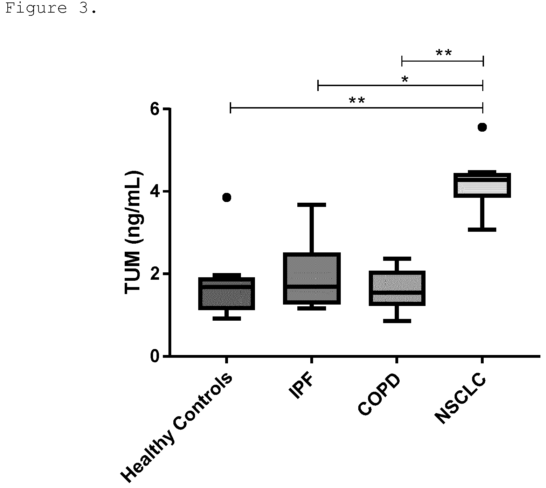

Cohort 1 consists of healthy controls and patients diagnosed with IPF, COPD and NSCLC, and the results are shown in Figure 3. Results from cohort 1 showed that TUM was significantly elevated in serum from NSCLC compared to healthy controls, IPF patients and COPD patients (p=0.007, p=0.03 and p=0.001 respectively). No significant difference was observed between healthy controls, IPF patients and COPD patients, which indicated that TUM may play a role in NSCLC, but not fibrotic lung disorders.

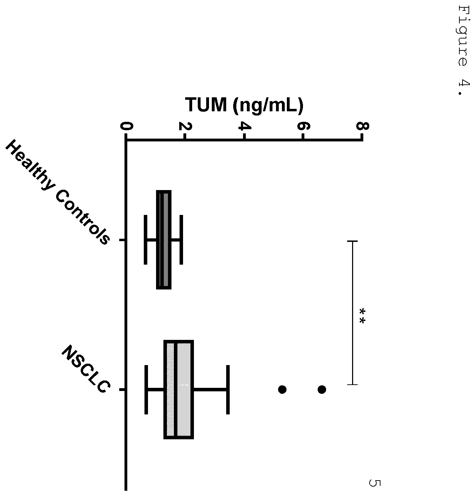

In cohort 2, TUM was measured in samples from healthy controls, and patients with NSCLC as shown in Figure 4. Here TUM was significantly upregulated in patients with NSCLC compared to healthy controls (p=0.002). There was no significant difference between the different cancer stages. The AUROC was used to evaluate the discriminative power of TUM in relation to NSCLC and healthy controls.

As shown in Table 5, TUM was able to discriminate between NSCLC patients and healthy controls in cohort 1 with an AUROC of 0.97, NSCLC patients and IPF patients with an AUROC 0.98 and NSCLC and COPD patients with an AUROC of 1.00. In cohort 2, TUM was able to identify NSCLC patients from healthy controls with an AUROC 0.73. These findings indicate that TUM levels are able to separate healthy controls from patients with NSCLC with a high diagnostic accuracy.

**Table 5. Discriminative performance of TUM in healthy controls and NSCLC.**

| | **Cut-off value (ng/mL)** | **Sensitivity** | **Specificity** | **AUROC (95% CI)** | **p-value** |
|---|---|---|---|---|---|
| **Cohort 1** | | | | | |
| NSCLC vs. healthy controls | 1. 97 | 100 | 87.5 | 0.97 (0.89-1.05) | 0.002 |
| NSCLC vs. IPF | 3.67 | 87.5 | 100 | 0.98 (0.75-1.00) | <0.0001 |
| NSCLC vs. COPD | 2.37 | 100 | 100 | 1.00 (0.79-1.00) | <0.0001 |
| | | | | | |

| **Cohort 2** | | | | | |
|---|---|---|---|---|---|
| NSCLC vs. healthy controls | 1.27 | 100 | 44 | 0.73 (0.60-0.84) | 0.003 |
| NSCLC stage IV vs. healthy controls | 1.27 | 100 | 60 | 0.87 (0.73-1.01) | 0.001 |

| | | | | | |
|---|---|---|---|---|---|
| NSCLC: non-small cell lung cancer, IPF: idiopathic pulmonary fibrosis, COPD: chronic obstructive pulmonary disease. | | | | | |

### Biological validation-TUM as a biomarker for CKD, SLE and LN

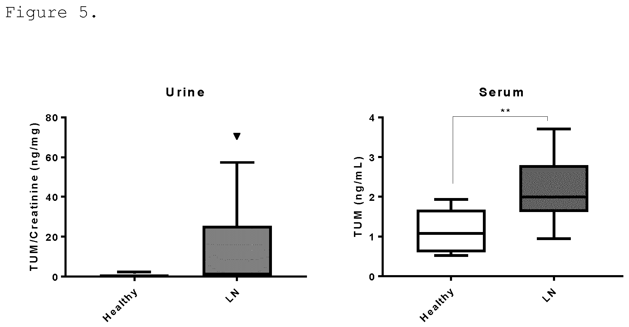

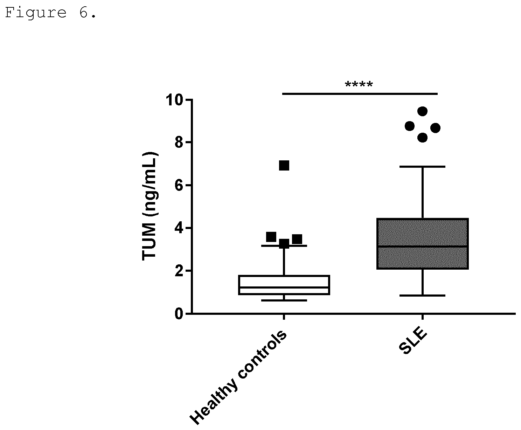

TUM was measured in two different patient cohorts; cohort 1 and cohort 2. In cohort 1, TUM was measured in serum and urine samples from healthy controls and patients with lupus nephritis (LN), and the results are shown in Figure 5. In cohort 2, TUM was measured in serum samples from healthy controls and patients with systemic lupus erythematosus (SLE, and the results are shown in Figure 6. Levels of TUM were found to be up to 2-fold upregulated in serum of patients with systemic lupus erythematosus (SLE) and lupus nephritis (LN) and 10-fold upregulated in urine of patients with LN.

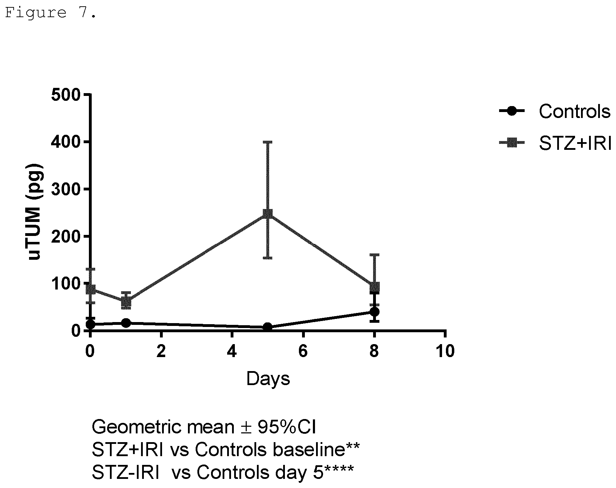

TUM was also measured in a rat rat model of diabetic kidney disease, the results of which are shown in Figure 7. Levels of TUM in the urine were found to be more elevated in diabetic rats (type 1 diabetes) compared to controls, and increased over time in the diabetic rats, with a peak 5 days after ischemic reperfusion injury (IRI).

### Conclusions

A novel competitive ELISA using a monoclonal antibody for detecting tumstatin has been developed (herein referred to as "TUM ELISA"). The assay was technically robust and specific towards the amino acid sequence PGLKGKRGDS (SEQ ID NO:1). The TUM fragment was detectable in human serum and urine, and was found to be significantly elevated in patients with NSCLC, compared to IPF, COPD and healthy controls; significantly elevated in patients with SLE or LN, compared to healthy controls; and significantly elevated in a rat model of diabetic kidney disease.

As shown herein, the TUM ELISA has a diagnostic potential within diagnosis of lung cancers, particularly NSCLC, and can separate these patients from patients with lung fibrosis. Based on the high diagnostic accuracy, this could be a biomarker of BM remodeling in lung cancer. Likewise, it has been shown herein that the TUM ELISA has a diagnostic potential within diagnosis of systemic lupus erythematosus (SLE), lupus nephritis (LN), and chronic kidney disease, particularly resulting from diabetes, SLE or LN.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'.

### References

1. Colorado PC, Torre A, Kamphaus G, et al. Anti-angiogenic cues from vascular basement membrane collagen. Cancer Res. 2000;60(9):2520-2526.
2. Pozzi A, Yurchenco PD, Iozzo R V. The nature and biology of basement membranes. Matrix Biol. 2017;57-58:1-11. doi:10.1016/j.matbio.2016.12.009.
3. Miner JH. Laminins and their roles in mammals. Microsc Res Tech. 2008;71(5):349-356. doi:10.1002/jemt.20563.
4. Sannes PL, Wang J. Basement membranes and pulmonary development. Exp Lung Res. 23(2):101-108. http://www.ncbi.nlm.nih.gov/pubmed/9088920. Accessed October 17, 2017.
5. Randles MJ, Humphries MJ, Lennon R. Proteomic definitions of basement membrane composition in health and disease. Matrix Biol. 2017;57-58:12-28. doi:10.1016/j.matbio.2016.08.006.
6. Gelse K. Collagens-structure, function, and biosynthesis. Adv Drug Deliv Rev. 2003;55(12):1531-1546. doi:10.1016/j.addr.2003.08.002.
7. Sand JM, Larsen L, Hogaboam C, et al. MMP mediated degradation of type IV collagen alpha 1 and alpha 3 chains reflects basement membrane remodeling in experimental and clinical fibrosis--validation of two novel biomarker assays. PLoS One. 2013;8(12):e84934. doi:10.1371/journal.pone.0084934.
8. Jarad G, Knutsen RH, Mecham RP, Miner JH. Albumin contributes to kidney disease progression in Alport syndrome. Am J Physiol - Ren Physiol. 2016;311(1):F120-F130. doi:10.1152/ajprenal.00456.2015.
9. Foster MH. Basement membranes and autoimmune diseases. Matrix Biol. 2017;57-58:149-168. doi:10.1016/J.MATBIO.2016.07.008.
10. Hamano Y, Zeisberg M, Sugimoto H, et al. Physiological levels of tumstatin, a fragment of collagen IV a3 chain, are generated by MMP-9 proteolysis and suppress angiogenesis via IVa3 integrin. Cancer Cell. 2003;3(6):589-601. doi:10.1016/S1535-6108(03)00133-8.
11. Sand JMB, Martinez G, Midjord A-K, Karsdal MA, Leeming DJ, Lange P. Characterization of serological neo-epitope biomarkers reflecting collagen remodeling in clinically stable chronic obstructive pulmonary disease. Clin Biochem. 2016;49(15):1144-1151. doi:10.1016/j.clinbiochem.2016.09.003.
12. Glentis A, Gurchenkov V, Matic Vignjevic D. Assembly, heterogeneity, and breaching of the basement membranes. Cell Adh Migr. 2014;8(3):236-245. http://www.ncbi.nlm.nih.gov/pubmed/24727304. Accessed October 17, 2017.
13. Eikesdal HP, Sugimoto H, Birrane G, et al. Identification of amino acids essential for the antiangiogenic activity of tumstatin and its use in combination antitumor activity. Proc Natl Acad Sci U S A. 2008;105:15040-15045. doi:10.1073/pnas.0807055105.
14. Gu Q, Zhang T, Luo J, Wang F. Expression, purification, and bioactivity of human tumstatin from Escherichia coli. Protein Expr Purif. 2006;47:461-466. doi:10.1016/j.pep.2006.01.011.
15. Hamano Y, Kalluri R. Tumstatin, the NC1 domain of α3 chain of type IV collagen, is an endogenous inhibitor of pathological angiogenesis and suppresses tumor growth. Biochem Biophys Res Commun. 2005;333:292-298. doi:10.1016/j.bbrc.2005.05.130.
16. Jiang C-P, Wu B-H, Chen S-P, et al. High COL4A3 expression correlates with poor prognosis after cisplatin plus gemcitabine chemotherapy in non-small cell lung cancer. Tumor Biol. 2013;34(1):415-420. doi:10.1007/s13277-012-0565-2.
17. Murphy SL, Xu J, Kochanek KD, Statistics V. National Vital Statistics Reports Deaths: Final Data for 2010. 2013;61(4).
18. Van der Velden J, Harkness LM, Barker DM, et al. The Effects of Tumstatin on Vascularity, Airway Inflammation and Lung Function in an Experimental Sheep Model of Chronic Asthma. Sci Rep. 2016;6:26309. doi:10.1038/srep26309.
19. Burgess JK, Boustany S, Moir LM, et al. Reduction of Tumstatin in Asthmatic Airways Contributes to Angiogenesis, Inflammation, and Hyperresponsiveness. Am J Respir Crit Care Med. 2010;181(2):106-115. doi:10.1164/rccm.200904-06310C.
20. Luo Y-Q, Yao L-J, Zhao L, Sun A-Y, Dong H, Du J-P, Wu S-Z, Hu W, Development of an ELISA for quantification of tumstatin in serum samples and tissue extracts of patients with lung carcinoma. Clin. Chim. Acta 2010;411:510-515. doi: 10.1016/j.cca.2010.01.001.
21. Revert F, Penadés JR, Plana M, Bernal D, Johansson C, Itarte E, Cervera J, Wieslander J, Quinone S, Saus J: "Phosphorylation of the Goodpasture Antigen by Type A Protein Kinases", The Journal Of Biological Chemistry, vol. 270, no. 2, 1 January 1995 (1995-01-01), pages 13254 - 13261, XP055566955 doi: http://dx.doi.org/10.1074/1bc.270.22.13254
22. Borza CM; Borza D-B, Pedchenko V, Saleem MA, Mathieson PW, Sado Y, Hudson HM, Pozzi A, Saus J, Abrahamson DR, Zent R, Hudson BG; "Human Podocytes Adhere to the KRGDS Motif of the 3 4 5 Collagen IV Network", Journal Of The American Society Of Nephrology, vol. 19, no. 4, 18 March 2008 (2008-03-18), US, pages 677 - 684, XP055567230, ISSN: 1046-6673, DOI: 10.1681/ASN.2007070793 DOI: http://dx.doi.org/10.1681/ASN.2007070793
23. Penadés JR , Bernal D, Revert F, Johansson C, Vicente J. Fresquet VJ, Cervera J, Wieslander J, Quinone S, Saus J: " Characterization and Expression of Multiple Alternatively Spliced Transcripts of the Goodpasture Antigen Gene Region", European Journal Of Biochemistry, vol. 229, no. 3, 1 May 1995 (1995-05-01), pages 754 - 760, XP000938485, ISSN: 0014-2956, DOI: 10.1111/J.1432-1033.1995.TB20524.X DOI: http://dx.doi.org/10.1111/j.1432-1033.1995.tb20524.x

## Claims

1. An immunoassay method for quantifying peptides having an N-terminus amino acid sequence PGLKGKRGDS (SEQ ID NO:1) in a patient biofluid sample, said method comprising contacting said patient biofluid sample with a monoclonal antibody specifically reactive with said N-terminus amino acid sequence PGLKGKRGDS (SEQ ID NO:1) wherein the monoclonal antibody does not specifically recognise or bind an N-extended elongated version of said N-terminus amino acid sequence or an N-truncated shortened version of said N-terminus amino acid sequence, and determining the amount of binding between said monoclonal antibody and said N-terminus amino acid sequence.

2. The method of claim 1 , wherein the patient biofluid sample is blood, urine, synovial fluid, serum or plasma.

3. A method of immunoassay for detecting lung cancer in a patient, the method comprising contacting a patient biofluid sample with a monoclonal antibody specifically reactive with an N-terminus amino acid sequence PGLKGKRGDS (SEQ ID NO:1) wherein the monoclonal antibody does not specifically recognise or bind an N-extended elongated version of said N-terminus amino acid sequence or an N-truncated shortened version of said N-terminus amino acid sequence, determining the amount of binding between said monoclonal antibody and peptides comprising said N-terminus amino acid sequence, and correlating said amount of binding with i) values associated with normal healthy subjects and/or ii) values associated with known lung cancer severity and/or iii) values obtained from said patient at a previous time point and/or iv) a predetermined cut-off value.

4. A method as claimed in claim 3, wherein the lung cancer is non-small cell lung cancer (NSCLC).

5. A method as claimed in claim 3 or 4, wherein the predetermined cut-off value is at least 2.00 ng/mL.

6. The method of any one of claims 3 to 5, wherein the patient biofluid sample is blood, urine, synovial fluid, serum or plasma.

7. A method of immunoassay for detecting chronic kidney disease (CKD) in a patient, the method comprising contacting a patient biofluid sample with a monoclonal antibody specifically reactive with an N-terminus amino acid sequence PGLKGKRGDS (SEQ ID NO:1) wherein the monoclonal antibody does not specifically recognise or bind an N-extended elongated version of said N-terminus amino acid sequence or an N-truncated shortened version of said N-terminus amino acid sequence, determining the amount of binding between said monoclonal antibody and peptides comprising said N-terminus amino acid sequence, and correlating said amount of binding with values associated with normal healthy subjects and/or values associated with known CKD severity and/or values obtained from said patient at a previous time point and/or a predetermined cut-off value.

8. A method as claimed in claim 7, wherein the chronic kidney disease is chronic kidney disease resulting from systemic lupus erythematosus, lupus nephritis or diabetes.

9. The method of claim or claim 8, wherein the patient biofluid sample is blood, urine, synovial fluid, serum or plasma.

10. A method of immunoassay for detecting systemic lupus erythematosus (SLE)_or lupus nephritis (LN) in a patient, the method comprising contacting a patient biofluid sample with a monoclonal antibody specifically reactive with an N-terminus amino acid sequence PGLKGKRGDS (SEQ ID NO:1) wherein the monoclonal antibody does not specifically recognise or bind an N-extended elongated version of said N-terminus amino acid sequence or an N-truncated shortened version of said N-terminus amino acid sequence, determining the amount of binding between said monoclonal antibody and peptides comprising said N-terminus amino acid sequence, and correlating said amount of binding with values associated with normal healthy subjects and/or values associated with known SLE or LN severity and/or values obtained from said patient at a previous time point and/or a predetermined cut-off value.

11. The method of claim 10, wherein the patient biofluid sample is blood, urine, synovial fluid, serum or plasma.

12. A monoclonal antibody specifically reactive with an N-terminus amino acid sequence PGLKGKRGDS (SEQ ID NO:1) wherein the monoclonal antibody does not specifically recognise or bind an N-extended elongated version of said N-terminus amino acid sequence or an N-truncated shortened version of said N-terminus amino acid sequence.

13. An assay kit comprising a monoclonal antibody specifically reactive with an N-terminus amino acid sequence PGLKGKRGDS (SEQ ID NO:1) wherein the monoclonal antibody does not specifically recognise or bind an N-extended elongated version of said N-terminus amino acid sequence or an N-truncated shortened version of said N-terminus amino acid sequence, and at least one of:
- a streptavidin coated well plate
- a biotinylated peptide PGLKGKRGDS-L-Biotin (SEQ ID NO:6), wherein L is an optional linker
- a secondary antibody for use in a sandwich immunoassay
- a calibrator peptide comprising the sequence PGLKGKRGDS (SEQ ID NO:1)
- an antibody biotinylation kit
- an antibody HRP labeling kit
- an antibody radiolabeling kit
- an assay visualization kit

14. An assay kit as claimed in claim 13, wherein the monoclonal antibody is raised against a synthetic peptide having the amino acid sequence PGLKGKRGDS (SEQ ID NO:1).

## Patentansprüche

1. Immunoassay-Verfahren zum Quantifizieren von Peptiden, die eine N-terminale Aminosäuresequenz PGLKGKRGDS (SEQ ID NO:1) in einer Probe von biologischem Fluid eines Patienten aufweisen, wobei das Verfahren das In-Kontakt-Bringen der Probe von biologischem Fluid eines Patienten mit einem monoklonalen Antikörper, der spezifisch auf die N-terminale Aminosäuresequenz PGLKGKRGDS reagiert (SEQ ID NO:1), wobei der monoklonale Antikörper eine N-erweiterte verlängerte Version der N-terminalen Aminosäuresequenz oder eine N-trunkierte verkürzte Version der N-terminalen Aminosäuresequenz nicht spezifisch erkennt oder bindet, und das Bestimmen des Betrags der Bindung zwischen dem monoklonalen Antikörper und der N-terminalen Aminosäuresequenz umfasst.

2. Verfahren nach Anspruch 1, wobei die Probe von biologischem Fluid eines Patienten Blut, Urin, Synovialflüssigkeit, Serum oder Plasma ist.

3. Immunoassay-Verfahren zum Detektieren von Lungenkrebs bei einem Patienten, wobei das Verfahren das In-Kontakt-Bringen einer Probe von biologischem Fluid eines Patienten mit einem monoklonalen Antikörper, der spezifisch auf die N-terminale Aminosäuresequenz PGLKGKRGDS reagiert (SEQ ID NO:1), wobei der monoklonale Antikörper eine N-erweiterte verlängerte Version der N-terminalen Aminosäuresequenz oder eine N-trunkierte verkürzte Version der N-terminalen Aminosäuresequenz nicht spezifisch erkennt oder bindet, und das Bestimmen des Betrags der Bindung zwischen dem monoklonalen Antikörper und Peptiden, welche die N-terminale Aminosäuresequenz umfassen, und das Korrelieren des Betrags der Bindung mit i) Werten, die normalen gesunden Personen zugeordnet sind, und/oder ii) Werten, die einem bekannten Schweregrad von Lungenkrebs zugeordnet sind, und/oder iii) Werten, die von dem Patienten an einem vorherigen Zeitpunkt erzielt wurden, und/oder iv) einem vorbestimmten Schwellenwert umfasst.

4. Verfahren nach Anspruch 3, wobei der Lungenkrebs ein nicht kleinzelliges Lungenkarzinom (NSCLC) ist.

5. Verfahren nach Anspruch 3 oder 4, wobei der vorbestimmte Schwellenwert mindestens 2,00 ng/mL beträgt.

6. Verfahren nach einem der Ansprüche 3 bis 5, wobei die Probe von biologischem Fluid eines Patienten Blut, Urin, Synovialflüssigkeit, Serum oder Plasma ist.

7. Immunoassay-Verfahren zum Detektieren einer chronischen Nierenerkrankung (CKD) bei einem Patienten, wobei das Verfahren das In-Kontakt-Bringen einer Probe von biologischem Fluid eines Patienten mit einem monoklonalen Antikörper, der spezifisch auf eine N-terminale Aminosäuresequenz PGLKGKRGDS reagiert (SEQ ID NO:1), wobei der monoklonale Antikörper eine N-erweiterte verlängerte Version der N-terminalen Aminosäuresequenz oder eine N-trunkierte verkürzte Version der N-terminalen Aminosäuresequenz nicht spezifisch erkennt oder bindet, das Bestimmen des Betrags der Bindung zwischen dem monoklonalen Antikörper und den Peptiden, welche die N-terminale Aminosäuresequenz umfassen, und das Korrelieren des Betrags der Bindung mit Werten, die normalen gesunden Personen zugeordnet sind, und/oder ii) Werten, die einem bekannten CKD-Schweregrad zugeordnet sind, und/oder iii) Werten, die von dem Patienten an einem vorherigen Zeitpunkt erzielt wurden, und/oder iv) einem vorbestimmten Schwellenwert umfasst.

8. Verfahren nach Anspruch 7, wobei die chronische Nierenerkrankung eine chronische Nierenerkrankung ist, die aus systemischem Lupus erythematodes, Lupusnephritis oder Diabetes entsteht.

9. Verfahren nach Anspruch 7 oder Anspruch 8, wobei die Probe von biologischem Fluid eines Patienten Blut, Urin, Synovialflüssigkeit, Serum oder Plasma ist.

10. Immunoassay-Verfahren zum Detektieren von systemischem Lupus erythematodes (SLE) oder Lupusnephritis (LN) bei einem Patienten, wobei das Verfahren das In-Kontakt-Bringen einer Probe von biologischem Fluid eines Patienten mit einem monoklonalen Antikörper, der spezifisch auf die N-terminale Aminosäuresequenz PGLKGKRGDS reagiert (SEQ ID NO:1), wobei der monoklonale Antikörper eine N-erweiterte verlängerte Version der N-terminalen Aminosäuresequenz oder eine N-trunkierte verkürzte Version der N-terminalen Aminosäuresequenz nicht spezifisch erkennt oder bindet, das Bestimmen des Betrags der Bindung zwischen dem monoklonalen Antikörper und Peptiden, welche die N-terminale Aminosäuresequenz umfassen, und das Korrelieren des Betrags der Bindung mit Werten, die normalen gesunden Personen zugeordnet sind, und/oder Werten, die einem bekannten SLE- oder LN- Schweregrad zugeordnet sind, und/oder Werten, die von dem Patienten an einem vorherigen Zeitpunkt erzielt wurden, und/oder einem vorbestimmten Schwellenwert umfasst.

11. Verfahren nach Anspruch 10, wobei die Probe von biologischem Fluid eines Patienten Blut, Urin, Synovialflüssigkeit, Serum oder Plasma ist.

12. Monoklonaler Antikörper, der mit einer N-terminalen Aminosäuresequenz PGLKGKRGDS spezifisch reagiert (SEQ ID NO:1), wobei der monoklonale Antikörper eine N-erweiterte verlängerte Version der N-terminalen Aminosäuresequenz oder eine N-trunkierte verkürzte Version der N-terminalen Aminosäuresequenz nicht spezifisch erkennt oder bindet.

13. Testkit, umfassend einen monoklonalen Antikörper, der spezifisch auf eine N-terminale Aminosäuresequenz PGLKGKRGDS reagiert (SEQ ID NO:1), wobei der monoklonale Antikörper eine N-erweiterte verlängerte Version der N-terminalen Aminosäuresequenz oder eine N-trunkierte verkürzte Version der N-terminalen Aminosäuresequenz und mindestens eines von:
- einer mit Streptavidin beschichteten Mikrotiterplatte,
- einem biotinylierten Peptid PGLKGKRGDS-L-Biotin (SEQ ID NO:6), wobei L ein optionaler Koppler ist,
- einem sekundären Antikörper zur Verwendung bei einem Sandwich-Immunoassay,
- einem Kalibrierpeptid, das die Sequenz PGLKGKRGDS umfasst (SEQ ID NO:1),
- einem Antikörper-Biotinylierungs-Kit,
- einem Antikörper-HRP-Markierungs-Kit,
- einem Kit zur radioaktiven Markierung von Antikörpern,
- einem Testvisualisierungskit
nicht spezifisch erkennt oder bindet.

14. Testkit nach Anspruch 13, wobei der monoklonale Antikörper gegen ein synthetisches Peptide gezüchtet wird, das die Aminosäuresequenz PGLKGKRGDS aufweist (SEQ ID NO:1).

## Revendications

1. Procédé de dosage immunologique permettant de quantifier des peptides présentant une séquence d'acides aminés N-terminale PGLKGKRGDS (SEQ ID NO:1) dans un échantillon de fluide biologique d'un patient, ledit procédé comprenant la mise en contact dudit échantillon de fluide biologique d'un patient avec un anticorps monoclonal spécifiquement réactif avec ladite séquence d'acides aminés N-terminale PGLKGKRGDS (SEQ ID NO:1), dans lequel l'anticorps monoclonal ne reconnaît ou ne se lie pas spécifiquement à une version prolongée étendue du côté N-terminal de ladite séquence d'acides aminés N-terminale ou à une version raccourcie tronquée côté N -terminal de ladite séquence d'acides aminés N-terminale, et la détermination du degré de liaison entre ledit anticorps monoclonal et ladite séquence d'acides aminés N-terminale.

2. Procédé selon la revendication 1, dans lequel l'échantillon de fluide biologique d'un patient est du sang, de l'urine, du fluide synovial, du sérum ou du plasma.

3. Procédé de dosage immunologique permettant de détecter un cancer du poumon chez un patient, le procédé comprenant la mise en contact d'un échantillon de fluide biologique d'un patient avec un anticorps monoclonal spécifiquement réactif avec une séquence d'acides aminés N-terminale PGLKGKRGDS (SEQ ID NO:1), dans lequel l'anticorps monoclonal ne reconnaît ou ne se lie pas spécifiquement à une version prolongée étendue du côté N-terminal de ladite séquence d'acides aminés N-terminale ou à une version raccourcie tronquée côté N-terminal de ladite séquence d'acides aminés N-terminale, la détermination du degré de liaison entre ledit anticorps monoclonal et des peptides comprenant ladite séquence d'acides aminés N-terminale, et la corrélation dudit degré de liaison avec i) des valeurs associées à des personnes normales en bonne santé, et/ou ii) des valeurs associées à la sévérité connue d'un cancer du poumon, et/ou iii) des valeurs obtenues dudit patient à un instant précédent, et/ou iv) une valeur seuil prédéterminée.

4. Procédé selon la revendication 3, dans lequel le cancer du poumon est un cancer du poumon non à petites cellules (NSCLC).

5. Procédé selon la revendication 3 ou 4, dans lequel la valeur seuil prédéterminée est au moins de 2,00 ng/mL.

6. Procédé selon l'une quelconque des revendications 3 à 5, dans lequel l'échantillon de fluide biologique d'un patient est du sang, de l'urine, du fluide synovial, du sérum ou du plasma.

7. Procédé de dosage immunologique permettant de détecter une insuffisance rénale chronique (CKD) chez un patient, le procédé comprenant la mise en contact d'un échantillon de fluide biologique d'un patient avec un anticorps monoclonal spécifiquement réactif avec une séquence d'acides aminés N-terminale PGLKGKRGDS (SEQ ID NO:1), dans lequel l'anticorps monoclonal ne reconnaît ou ne se lie pas spécifiquement à une version prolongée étendue du côté N-terminal de ladite séquence d'acides aminés N-terminale ou à une version raccourcie tronquée côté N -terminal de ladite séquence d'acides aminés N-terminale, la détermination du degré de liaison entre ledit anticorps monoclonal et des peptides comprenant ladite séquence d'acides aminés N-terminale, et la corrélation dudit degré de liaison avec des valeurs associées à des personnes normales en bonne santé, et/ou des valeurs associées à la sévérité connue d'un cancer du poumon, et/ou des valeurs obtenues dudit patient à un instant précédent, et/ou une valeur seuil prédéterminée.

8. Procédé selon la revendication 7, dans lequel l'insuffisance rénale chronique est une insuffisance rénale chronique résultant d'un lupus érythémateux disséminé, d'un lupus néphrétique ou d'un diabète.

9. Procédé selon la revendication 7 ou la revendication 8, dans lequel l'échantillon de fluide biologique d'un patient est du sang, de l'urine, du fluide synovial, du sérum ou du plasma.

10. Procédé de dosage immunologique permettant de détecter un lupus érythémateux disséminé (SLE) ou un lupus néphrétique (LN) chez un patient, le procédé comprenant la mise en contact d'un échantillon de fluide biologique d'un patient avec un anticorps monoclonal spécifiquement réactif avec une séquence d'acides aminés N-terminale PGLKGKRGDS (SEQ ID NO:1), dans lequel l'anticorps monoclonal ne reconnaît ou ne se lie pas spécifiquement à une version prolongée étendue du côté N-terminal de ladite séquence d'acides aminés N-terminale ou à une version raccourcie tronquée côté N-terminal de ladite séquence d'acides aminés N-terminale, la détermination du degré de liaison entre ledit anticorps monoclonal et des peptides comprenant ladite séquence d'acides aminés N-terminale, et la corrélation dudit degré de liaison avec des valeurs associées à des personnes normales en bonne santé, et/ou ii) des valeurs associées à une sévérité connue de SLE ou LN, et/ou des valeurs obtenues dudit patient à un instant précédent, et/ou une valeur seuil prédéterminée.

11. Procédé selon la revendication 10, dans lequel l'échantillon de fluide biologique d'un patient est du sang, de l'urine, du fluide synovial, du sérum ou du plasma.

12. Anticorps monoclonal spécifiquement réactif avec une séquence d'acides aminés N-terminale PGLKGKRGDS (SEQ ID NO:1), dans lequel l'anticorps monoclonal ne reconnaît ou ne se lie pas spécifiquement à une version prolongée étendue du côté N-terminal de ladite séquence d'acides aminés N-terminale ou à une version raccourcie tronquée côté N-terminal de ladite séquence d'acides aminés N-terminale.

13. Kit d'immunoessai, comprenant un anticorps monoclonal spécifiquement réactif avec une séquence d'acides aminés N-terminale PGLKGKRGDS (SEQ ID NO:1), dans lequel l'anticorps monoclonal ne reconnaît ou ne se lie pas spécifiquement à une version prolongée étendue du côté N-terminal de ladite séquence d'acides aminés N-terminale ou une version raccourcie tronquée côté N-terminal de ladite séquence d'acides aminés N-terminale, et à au moins un élément parmi :
- une plaque de microtitration recouverte de streptavidine,
- un peptide biotinylé PGLKGKRGDS-L-Biotine (SEQ ID NO:6), dans lequel L est un lieur en option,
- un anticorps secondaire à utiliser dans une immunoanalyse « en sandwich »,
- un peptide calibreur comprenant la séquence PGLKGKRGDS (SEQ ID NO:1),
- un kit de biotinylation d'anticorps,
- un kit de marquage HRP d'anticorps,
- un kit de radiomarquage d'anticorps,
- un kit de visualisation d'immunoessai.

14. Kit d'immunoessai selon la revendication 13, dans lequel l'anticorps monoclonal est dirigé contre un peptide synthétique présentant une séquence d'acides aminés PGLKGKRGDS (SEQ ID NO:1).
